# EUROPEAN PATENT APPLICATION

(11) **EP 4 066 753 A2**
(43) Date of publication of application: **05.10.2022**
(21) Application number: 22165295.1
(22) Date of filing: 30.03.2022
(51) Int. Cl.: A61B 17/12

(54) **BIO-ABSORBABLE COILED FIBER**

(30) Priority: 31.03.2021 US 202117218968
(71) Applicant: DePuy Synthes Products, Inc., Raynham, MA 02767 (US)
(72) Inventor: SLAZAS, Robert, Raynham, 02767 (US)
(74) Representative: Carpmaels & Ransford LLP

(57) **Abstract**

An implant can be placed into an aneurysm sac that includes a bioabsorbable coiled fiber having a coiled shape similar to a metallic embolic coil. The implant can have an absorption rate long enough so that the aneurysm can become sufficiently occluded and sufficiently healed as the implant is absorbed. The implant can include crossing microfibers to initiate thrombosis within the aneurysm and/or a stretch-resistant member extending through a lumen of the coiled shape to inhibit elongation of the coiled shape. The crossing microfibers can be attached to the coiled fiber by friction fit between coil windings, heat bonding to the coiled fiber, and/or securement to the stretch-resistant member. The coiled fiber, crossing microfibers, and stretch-resistant member can have rates of absorption that differ to promote an accelerated sequence of occlusion and healing of the aneurysm.

## Description

### FIELD

The present invention generally relates to implantable medical devices, and more particularly to bio-absorbable implants to induce venous stasis.

### BACKGROUND

Aneurysms can be intravascularly treated by delivering a treatment device to the aneurysm to fill the sac of the aneurysm with embolic material and/or block the neck of the aneurysm to inhibit blood flow into the aneurysm. When filling the aneurysm sac, the embolic material can promote blood clotting to create a thrombotic mass within the aneurysm. When treating the aneurysm neck without substantially filling the aneurysm sac, blood flow into the neck of the aneurysm can be inhibited to induce venous stasis in the aneurysm and facilitate natural formation of a thrombotic mass within the aneurysm.

In some current treatments, multiple metal-based (e.g. platinum) embolic coils are used to either fill the aneurysm sac or treat the entrance of the aneurysm neck. When the aneurysm sac is packed with embolic coils, the aneurysm typically takes on the shape of the metal coils packed within it, making the aneurysm a permanent volume within the skull. Placing embolic coils exclusively at the aneurysm neck can allow thrombus to form naturally within the aneurysm sac and allow the sac to shrink in volume; however, such placement is difficult without filling some or all of the aneurysm sac. In some instances, when either the aneurysm sac is packed with embolic coils or the neck is treated, blood flow can resume into the aneurysm following an initial treatment, requiring a subsequent treatment where additional embolic coils or treatment devices are placed to treat the aneurysm, further increasing the volume of metal at the aneurysm, and possibly extending the aneurysm sac.

### SUMMARY

Examples presented herein generally include placing an at least partially bioabsorbable implant into an aneurysm sac. The implant includes a bioabsorbable coiled fiber having a coiled shape similar to a metallic embolic coil. The implant can include an absorption rate long enough so that the aneurysm can become sufficiently occluded and sufficiently healed as the implant is absorbed. The implant can include crossing microfibers to initiate thrombosis within the aneurysm and/or a stretch-resistant member extending through a lumen of the coiled shape to inhibit elongation of the coiled shape. The crossing microfibers can be attached to the coiled fiber by friction fit between coil windings, heat bonding to the coiled fiber, and/or securement to the stretch-resistant member. The coiled fiber, crossing microfibers, and stretch-resistant member can have rates of absorption that differ to promote an accelerated sequence of occlusion and healing of the aneurysm.

An example occlusive device can include a fiber in a coiled configuration (coiled fiber) and crossing microfibers. The coiled fiber can include a first bioabsorbable material composition. The crossing microfibers can be mounted on at least a portion of the coiled fiber, can extend radially from an outer diameter of the coiled fiber, and can include a second bioabsorbable material composition.

At least a portion of the crossing microfibers can be secured to the coiled fiber by at least one of heat, glue, solvent bonding, mechanical wrapping, interference fit, or friction fit.

The crossing microfibers can be mounted on the coiled fiber across a proximal portion of the coiled fiber that has a length measuring from about one half to about one third of a total length of the coiled fiber.

The occlusive device can further include a stretch-resistant member positioned within a coiled fiber lumen of the coiled fiber. The stretch-resistant member can be secured at a distal end of the coiled fiber and secured at a proximal end of the coiled fiber. The stretch-resistant member can include a third bioabsorbable material composition.

The first bioabsorbable material composition can be distinct from the second and third bioabsorbable material compositions. The second bioabsorbable composition can be distinct from the first and third bioabsorbable material compositions. The third bioabsorbable composition can be distinct from the first and the second bioabsorbable material compositions.

The second bioabsorbable material composition can have an absorption rate higher than an absorption rate of the first bioabsorbable material composition.

The first and second bioabsorbable material compositions can each respectively include a material selected from a group consisting of polyethylbenzene, polydimethylsiloxane, polyglycolic acid, poly-L-lactic acid, polycaprolactive, polyhydroxybutyrate, polyhydroxyvalerate, polydioxanone, polycarbonate, polyanhydride, polycaprolactone, polydioxanone, polybutyrolactone, polyvalerolactone, poly(lactic-co-glycolic acid), cellulose acetate propionate, and combinations thereof.

The occlusive device can be entirely bioabsorbable, i.e. including only bioabsorbable materials.

The coiled fiber can have a fiber diameter from about 0.001 inches to about 0.003 inches.

The outer diameter of the coiled configuration of the coiled fiber can measure from about 0.008 inches to about 0.018 inches.

A majority of the crossing microfibers can each respectively have a diameter from about 0.0002 inches to about 0.001 inches.

A distal portion of the coiled fiber can be configured to form an outer perimeter arrangement within an aneurysm. A proximal portion of the coiled fiber can be configured to form an interior array within the outer perimeter arrangement.

Another example occlusive device can include a fiber in a coiled configuration (coiled fiber), a stretch-resistant member, and crossing microfibers. The stretch-resistant member can be positioned within a coiled fiber lumen of the coiled fiber. The crossing microfibers can be securely attached to the stretch-resistant member. The crossing microfibers can extend radially from an outer diameter of the coiled fiber.

The crossing microfibers can extend radially from the coiled fiber across a proximal portion of the coiled fiber such that the crossing microfibers are absent from a distal portion of the coiled fiber, the proximal portion of the coiled fiber has a length measuring from about one half to one third of a total length of the coiled fiber, and the distal portion of the coiled fiber has a length measuring from about one half to about two thirds of the total length of the coiled fiber.

The distal portion of the coiled fiber can be configured to form an outer perimeter arrangement within an aneurysm. The proximal portion of the coiled fiber can be configured to form an interior array within the outer perimeter arrangement.

The occlusive device can be entirely bioabsorbable, i.e. including only bioabsorbable materials.

The coiled fiber can include a first bioabsorbable material composition having a first absorption rate. The crossing microfibers can include a second bioabsorbable material having a second absorption rate. The stretch-resistant member can include a third bioabsorbable material composition having a third absorption rate. The second absorption rate can be higher than the first absorption rate and the third absorption rate.

An example method for treating an aneurysm can include the following steps performed in a variety of orders and together with additional steps as understood by a person skilled in the pertinent art. An occlusive device can be delivered intravascularly to an aneurysm. A distal portion of a coiled fiber of the occlusive device can be positioned into a sac of the aneurysm such that the distal portion forms an outer perimeter arrangement within the aneurysm sac. A proximal portion of the coiled fiber can be positioned within the outer perimeter arrangement such that crossing microfibers extending from an outer diameter of the coiled fiber and positioned along the proximal portion of the coiled fiber are inhibited from crossing a neck of the aneurysm by the outer perimeter arrangement.

The example method can further include allowing at least a portion of the occlusive device to be absorbed into living tissue.

The example method can further include allowing the crossing microfibers to be absorbed into the living tissue at an absorption rate that is higher than an absorption rate of the coiled fiber.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
Figure 1 illustrates a cut-away view of an example implant according to aspects of the present invention.
Figure 2A illustrates a cut-away view of another example implant according to aspects of the present invention.
Figures 2B and 2C illustrate cross-sectional views of the example implant illustrated in Figure 2A.
Figure 3A illustrates a cross-sectional view of another example implant according to aspects of the present invention.
Figures 3B and 3C illustrate cross-sectional views of the example implant illustrated in Figure 3A.
Figure 4A illustrates formation of an outer perimeter arrangement within an aneurysm according to aspects of the present invention.
Figure 4B illustrates formation of an interior array within the outer perimeter arrangement of Figure 4A according to aspects of the present invention.
Figure 4C illustrates an enlarged view of the interior array of Figure 4B.
Figures 5A through 5C are a sequence of illustrations depiction healing and occlusion of the aneurysm and absorption of an example implant according to aspects of the present invention.
Figure 6 is a flow diagram outlining steps of aneurysm treatment according to aspects of the present invention.

### DETAILED DESCRIPTION

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

As used herein, the term "absorption rate" is a comparative property of bioabsorbable materials and of bioabsorbable structures. A bioabsorbable material having a higher absorption rate is absorbed into tissue more quickly than a comparison bioabsorbable material given that the conditions under which the two bioabsorbable materials are being absorbed are equal (e.g. geometry, surrounding environment, etc.). A bioabsorbable structure having a higher absorption rate maintains structural integrity for a shorter period of time than a comparison bioabsorbable structure in an equivalent environment; the compared bioabsorbable structures can have the same or distinct material composition and the same or different geometry.

As used herein, the term "absorption time" refers to a length of time a bioabsorbable structure maintains structural integrity while in an environment in which the structure is being absorbed.

As used herein, the term "bioabsorbable" means capable of being absorbed into tissue or bodily fluids, particularly living tissue and similar definitions as understood by persons skilled in the pertinent art.

As used herein, the terms "tubular" and "tube" are to be construed broadly and are not limited to a structure that is a right cylinder or strictly circumferential in cross-section or of a uniform cross-section throughout its length. For example, a tubular structure or system is generally illustrated as a substantially right cylindrical structure. However, the tubular system may have a tapered or curved outer surface without departing from the scope of the present disclosure.

Examples presented herein generally include placing an at least partially bioabsorbable implant including a bioabsorbable coiled fiber into an aneurysm sac. The bioabsorbable coiled fiber can have a coiled shape similar to a metallic embolic coil. A potential advantage of the coiled shape is that in some examples, the coiled fiber can be delivered using delivery systems suitable for delivering an embolic coil as understood by a person skilled in the pertinent art. The implant can include crossing microfibers to initiate thrombosis within the aneurysm sac. Another potential advantage of the coiled shape is that crossing microfibers may be held between windings of the coiled fiber by interference fit or friction fit. The implant can include a stretch-resistant member extending through a lumen of the coiled shape to inhibit elongation of the coiled shape. The crossing microfibers can be attached to the coiled fiber by friction fit between coil windings, heat bonding to the coiled fiber, and/or securement to the stretch-resistant member.

The implant can have an absorption rate long enough so that the aneurysm can become sufficiently occluded and sufficiently healed as the implant is absorbed. The coiled fiber, crossing microfibers, and stretch-resistant member can have rates of absorption that differ to promote an accelerated sequence of occlusion and healing of the aneurysm.

Bioabsorbable materials suitable for the implant can include, but are not limited to polyethylbenzene, polydimethylsiloxane, polyglycolic acid, poly-L-lactic acid, polycaprolactive, polyhydroxybutyrate, polyhydroxyvalerate, polydioxanone, polycarbonate, polyanhydride, polycaprolactone, polydioxanone, polybutyrolactone, polyvalerolactone, poly(lactic-co-glycolic acid), cellulose acetate propionate, and combinations thereof. Material composition of component parts of the implant can be selected to achieve a desired absorption rate/time for the respective component parts. Geometry can also affect absorption rate/time such that two component parts having the same material composition and differing geometry can be absorbed at different absorption rates/times. For instance, when the crossing microfibers and the coiled fiber have identical material compositions, the crossing microfibers may be absorbed in a shorter absorption time compared to the coiled fiber due to a larger ratio of surface area to volume of the crossing microfibers.

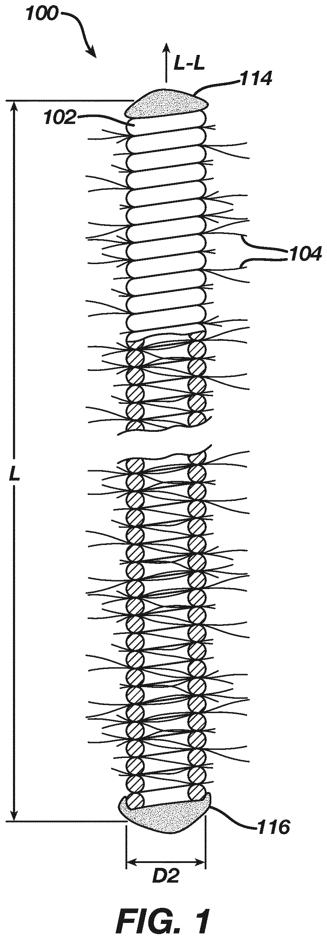

Figure 1 illustrates a cut-away view of an example implant 100. The implant 100 includes a coiled fiber 102 and crossing microfibers 104 extending radially from an outer diameter D2 of the coiled shape of the coiled fiber 102. The coiled fiber 102 can have a first bioabsorbable material composition, and the crossing microfibers 104 can have a second bioabsorbable material composition that is the same as or distinct from the first bioabsorbable material. The first and second bioabsorbable materials can have the same absorption rate or different absorption rates.

Preferably, the crossing microfibers 104 have a shorter absorption time and higher absorption rate than the coiled fiber 102. The second bioabsorbable material composition of the crossing microfibers 104 can have an absorption rate higher than an absorption rate of the first bioabsorbable material composition of the coiled fiber 102. The absorption time/rate of the crossing microfibers can be selected to promote cellular and/or metabolic activity within the aneurysm sac. The absorption time/rate of the coiled fiber 102 can be selected to maintain structural integrity of the coil, for example to inhibit migration of the crossing microfibers from the aneurysm during healing and occlusion and/or to inhibit ingress of blood into the aneurysm sac during aneurysm occlusion and healing.

The crossing microfibers 104 can include a material that is an irritant to promote more rapid cellular response and accelerate healing of the aneurysm. For instance, the crossing microfibers 104 can include a bioabsorbable material in combination with a steroidal anti-inflammatory drug, a nonsteroidal anti-inflammatory drug, an angiogenic drug, or any suitable irritant that can result in a foreign body response or cellular response. The crossing microfibers 104 can be substantially composed of an irritant. Alternatively, the crossing microfibers 104 can be imbedded or coated with an irritant. The crossing microfibers 104 can be secured to the coiled fiber 102 by heat, glue, solvent bonding, mechanical wrapping, interference fit, or friction fit.

The coiled fiber 102 extends along a longitudinal axis L-A and has a length L measured between a proximal end 116 and a distal end 114 of the coiled shape. As illustrated, the coiled fiber 102 can be capped at the proximal end 116 and the distal end 114. The distal end 114 can be bare or have an atraumatic cap as understood by a person skilled in the pertinent art. The proximal end 116 can include a detachment feature configured to interface with an implant delivery system, for example a delivery system suitable for delivering a metallic embolic coil, a variation thereof, or an alternative thereto as understood by a person skilled in the pertinent art.

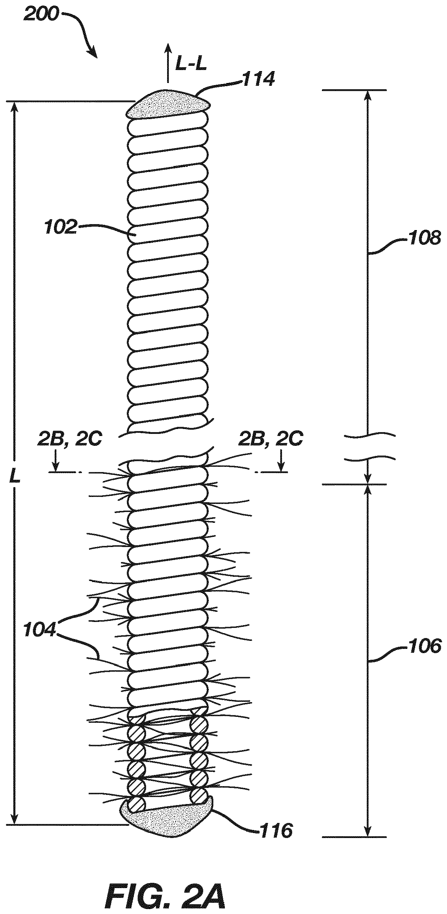

Figure 2A illustrates a cut-away view of another example implant 200 similar to the implant 100 illustrated in Figure 1 but for absence of crossing microfibers 104 on a distal portion 108 of the coiled fiber 102. The proximal portion 106 can include crossing microfibers 104 distributed along its entire length. The proximal portion 106 can have a length measuring from about one half to about one third of the total length L of the coiled fiber 102. Correspondingly, the distal portion can have a length measuring from about one half to about two thirds of the total length L of the coiled fiber 102.

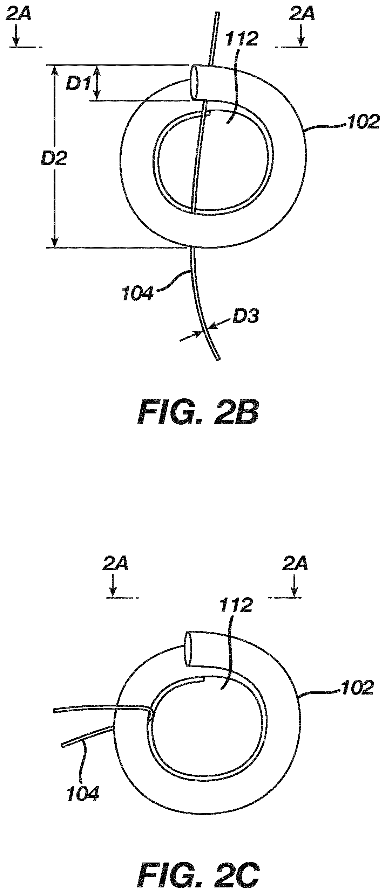

Figures 2B and 2C illustrate cross-sectional views of the example implant 200 as indicated in Figure 2A. Crossing microfibers 104 can extend across a lumen 112 of the coiled shape of the coiled fiber 102, exiting the coiled shape on opposite sides as illustrated in Figure 2B. The implant 200 can additionally, or alternatively, include crossing microfibers 104 that bend about 180° around a winding of the coil such that both ends of the crossing microfiber 104 exit the same side of the coiled shape as illustrated in Figure 2C. The implant 200 may have a combination of crossing microfibers 104 extending across a lumen 112, bending around a winding of the coil, and other variations thereof, or alternatives thereto as understood by a person skilled in the pertinent art.

The coiled fiber 102 can have a diameter D1 from about 0.001 inches (about 25 micrometers) to about 0.003 inches (about 76 micrometers). The outer diameter D2 of the coiled configuration of the coiled fiber 102 can measure from about 0.008 inches (about 200 micrometers) to about 0.018 inches (about 460 micrometers). A majority of the crossing microfibers 104 can each respectively have a diameter D3 from about 0.0002 inches (about 5 micrometers) to about 0.001 inches (about 25 micrometers).

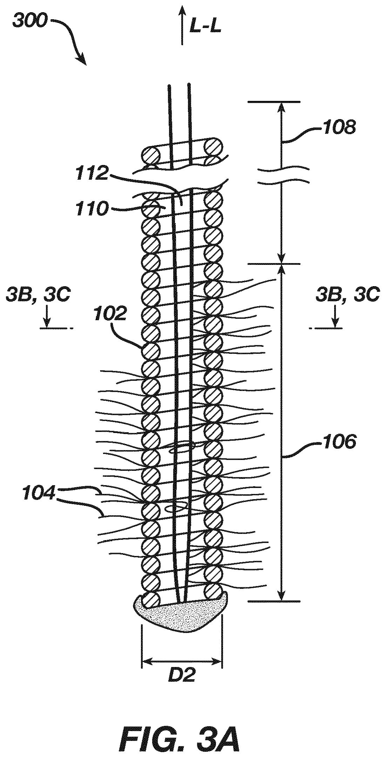

Figure 3A illustrates a cross-sectional view of another example implant 300 including a stretch-resistant member 110 within the lumen 112 of the coiled fiber 102. The stretch-resistant member 110 can include a fiber, tube, or other structure that is resistant to elongating when a longitudinal force is applied. The stretch-resistant member 110 can be secured at the distal end 114 of the coiled fiber 102 and secured at the proximal end 116 of the coiled fiber 102. The stretch-resistant member can include a third bioabsorbable material composition. The third bioabsorbable material can have an absorption rate that is the same as or distinct from the first bioabsorbable material. The absorption rate of the third bioabsorbable material can be the same or distinct from the second bioabsorbable material. The absorption time/rate of the stretch-resistant member 110 can be selected to maintain structural integrity of the coiled fiber 102 and/or crossing microfibers 104, for example to inhibit migration of the crossing microfibers from the aneurysm during healing and occlusion and/or to inhibit ingress of blood into the aneurysm sac during aneurysm occlusion and healing.

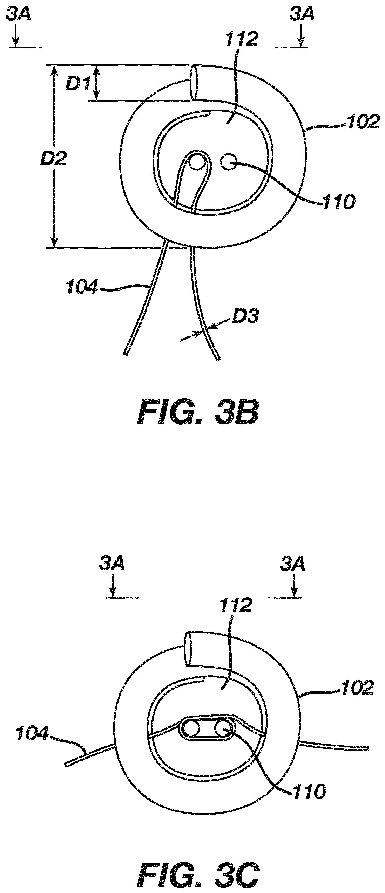

Figures 3B and 3C illustrate cross-sectional views of the example implant 300 illustrated in Figure 3A. The crossing microfibers 104 can be wrapped around the stretch-resistant member 110 in multiple configurations, including those illustrated in Figures 3B and 3C and variations thereof as understood by a person skilled in the pertinent art. The implant 300 can include crossing microfibers 104 having ends that exit the same side of the coiled shape as illustrated in Figure 3B, crossing microfibers having ends that exit opposite sides of the coiled shape as illustrated in Figure 3C, and/or crossing microfibers having ends that exit the coiled shape at an arbitrary angle to each other. The crossing microfibers can be attached to the stretch-resistant member such that only one end of the crossing microfiber exits the lumen of the coiled shape.

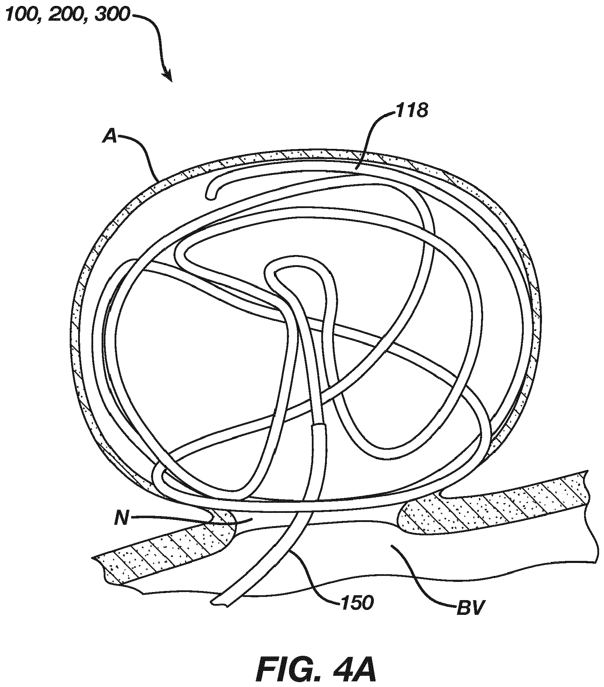

Figures 4A through 4C illustrate treatment of an aneurysm A with an example occlusive device configured similarly to the implants 100, 200, 300 disclosed herein. Preferably, the occlusive device lacks crossing microfibers on the distal portion 108 of the implant similar to the implants 200, 300 illustrated in Figures 2A through 3C. A delivery tube 150 can be positioned at the neck N of the aneurysm A and/or within the sac of the aneurysm A and the implant 200, 300 can be moved distally through the delivery tube 150 into the aneurysm sac.

Figure 4A illustrates formation of an outer perimeter arrangement 118 within an aneurysm A. A distal portion of the implant 200, 300 winds within the aneurysm sac as the implant 200, 300 is moved distally out of the delivery tube 150 so that the coiled fiber 102 forms a crisscrossing network at the wall of the aneurysm A and across the neck N of the aneurysm A. As density of coiled fiber 102 crisscrossed across the neck N of the aneurysm A increases, the perimeter arrangement 118 forms a barrier across the aneurysm neck N inhibiting the proximal portion 106 of the implant 200, 300 from exiting the aneurysm sac.

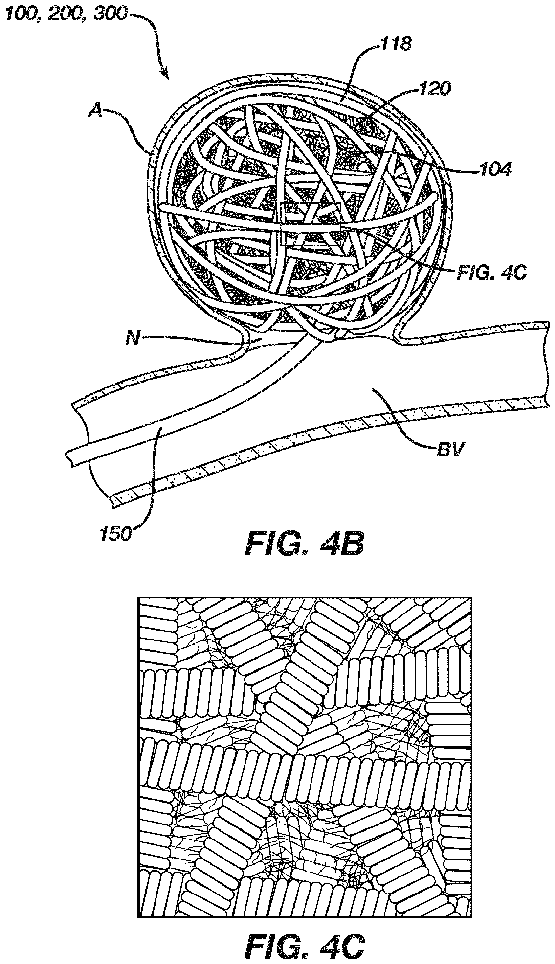

Additionally, or alternatively, a first implant can be implanted to form the outer perimeter arrangement 118 illustrated in Figure 4A. The outer perimeter arrangement 118 may form a barrier across the neck N of the aneurysm as understood by a person skilled in the pertinent art. The implant 100, as illustrated in Figure 1, can have crossing microfibers 104 along the length L of the coiled fiber 102 and can follow the outer perimeter and form an interior array 120 similar to as illustrated in Figure 4B.

Figure 4B illustrates formation of an interior array 120 within the outer perimeter arrangement 118 illustrated in Figure 4A. Figure 4C illustrates an enlarged view of the interior array of Figure 4B. The interior array 120 includes coiled fiber 102 having crossing microfibers 104 extending radially therefrom. The crossing microfibers 104 are inhibited by the outer perimeter arrangement 118 from crossing the neck N of the aneurysm A into the adjacent blood vessel BV.

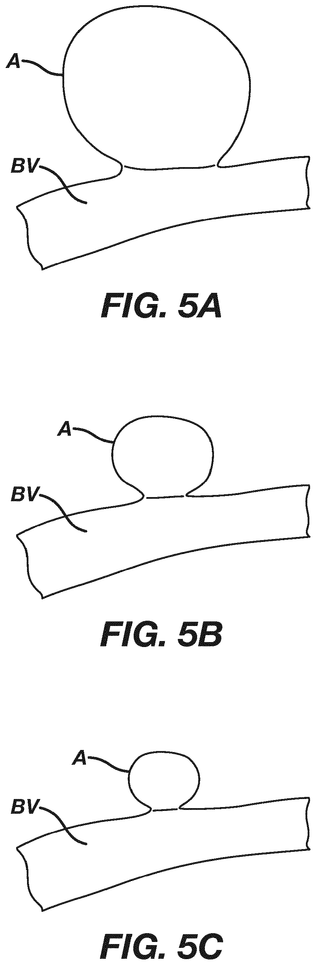

Figures 5A through 5C are a sequence of illustrations depicting healing and occlusion of the aneurysm and absorption of an example occlusive device. The occlusive device can be implanted similar to as illustrated in Figures 4A through 4C and variations thereof as understood by a person skilled in the pertinent art. The occlusive device can be configured similar to implants 100, 200, 300 disclosed herein and variations thereof as understood by a person skilled in the pertinent art. The sequence of illustrations in Figures 5A through 5C show the occlusive device 100, 200, 300 shrinking in size as it is absorbed into tissue. As the occlusive device 100, 200, 300 shrinks in size, the volume of the aneurysm A also shrinks. The crossing microfibers 104 can promote rapid thrombosis in the sac of the aneurysm. The coiled fiber 102 can maintain structural integrity of the implant 100, 200, 300 as the aneurysm A heals. An overwhelming majority of the crossing microfibers 104 can be absorbed before a majority of the coiled fiber 102 is absorbed.

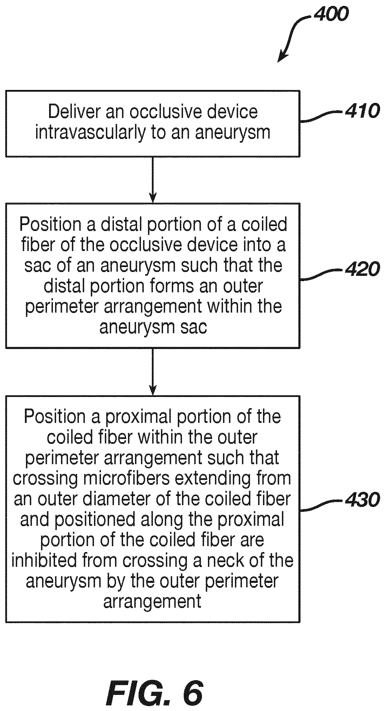

Figure 6 is a flow diagram outlining steps of a method 400 for aneurysm treatment.

At step 410, an occlusive device can be delivered intravascularly to an aneurysm. The occlusive device can be configured similar to occlusive devices and implants 100, 200, 300 disclosed herein, variations thereof, and alternatives thereto as understood by a person skilled in the pertinent art.

At step 420, a distal portion of a coiled fiber of the occlusive device can be positioned into a sac of the aneurysm such that the distal portion forms an outer perimeter arrangement within the aneurysm sac. The coiled fiber can be configured similar to coiled fiber 102 disclosed herein, variations thereof, and alternatives thereto as understood by a person skilled in the pertinent art. The resulting outer perimeter arrangement can be configured similar to the outer perimeter arrangement 118 illustrated in Figures 4A and 4B, variations thereof, and alternatives thereto as understood by a person skilled in the pertinent art.

At step 430, a proximal portion 106 of the coiled fiber can be positioned within the outer perimeter arrangement such that crossing microfibers extending from an outer diameter of the coiled fiber and positioned along the proximal portion of the coiled fiber are inhibited from crossing a neck of the aneurysm by the outer perimeter arrangement. The crossing microfibers can be configured similar to crossing microfibers 104 disclosed herein, variations thereof, and alternatives thereto as understood by a person skilled in the pertinent art.

The method 400 can proceed by allowing at least a portion of the occlusive device to be absorbed into living tissue. The crossing microfibers can be absorbed into the living tissue at an absorption rate that is higher than an absorption rate of the coiled fiber.

The descriptions contained herein are examples of embodiments of the invention and are not intended to limit the scope of the invention. As described herein, the invention contemplates many variations and modifications of an occlusive device and methods of treating an implant, including alternative mechanical constructions, alternative geometries, alternative material selections, ancillary treatment steps, etc. Modifications and variations apparent to those having skilled in the pertinent art according to the teachings of this disclosure are intended to be within the scope of the claims which follow.

### ASPECTS OF THE INVENTION

1. A method for treating an aneurysm, the method comprising:
   delivering an occlusive device intravascularly to an aneurysm;
   positioning a distal portion of a coiled fiber of the occlusive device into a sac of the aneurysm such that the distal portion forms an outer perimeter arrangement within the aneurysm sac; and
   positioning a proximal portion of the coiled fiber within the outer perimeter arrangement such that crossing microfibers extending from an outer diameter of the coiled fiber and positioned along the proximal portion of the coiled fiber are inhibited from crossing a neck of the aneurysm by the outer perimeter arrangement.
2. The method of aspect 1, further comprising:
   allowing at least a portion of the occlusive device to be absorbed into living tissue.
3. The method of aspect 2, further comprising:
   allowing the crossing microfibers to be absorbed into the living tissue at an absorption rate that is higher than an absorption rate of the coiled fiber.

## Claims

1. An occlusive device comprising:
a fiber in a coiled configuration, the coiled fiber comprising a first bioabsorbable material composition; and
a plurality of crossing microfibers mounted on at least a portion of the coiled fiber, extending radially from an outer diameter of the coiled fiber, and comprising a second bioabsorbable material composition.

2. The occlusive device of claim 1, wherein at least a portion of the plurality of crossing microfibers are secured to the coiled fiber by at least one of heat, glue, solvent bonding, mechanical wrapping, interference fit, or friction fit.

3. The occlusive device of claim 1,
wherein the plurality of crossing microfibers are mounted on the coiled fiber across a proximal portion of the coiled fiber, and
wherein the proximal portion comprises a length measuring from about one half to about one third of a total length of the coiled fiber.

4. The occlusive device of claim 1, further comprising a stretch-resistant member positioned within a coiled fiber lumen of the coiled fiber, secured at a distal end of the coiled fiber, secured at a proximal end of the coiled fiber, and comprising a third bioabsorbable material composition.

5. The occlusive device of claim 4,
wherein the first bioabsorbable material composition is distinct from the second and third bioabsorbable material compositions,
wherein the second bioabsorbable composition is distinct from the first and third bioabsorbable material compositions, and
wherein the third bioabsorbable composition is distinct from the first and the second bioabsorbable material compositions.

6. The occlusive device of claim 1, wherein the second bioabsorbable material composition comprises an absorption rate higher than an absorption rate of the first bioabsorbable material composition.

7. The occlusive device of claim 1, wherein the first and second bioabsorbable material compositions each respectively comprises a material selected from a group consisting of polyethylbenzene, polydimethylsiloxane (PDMS), polyglycolic acid (PGA), poly-L-lactic acid (PLA), polycaprolactive, polyhydroxybutyrate, polyhydroxyvalerate, polydioxanone, polycarbonate, polyanhydride, polycaprolactone (PCL), polydioxanone (PDO), polybutyrolactone (PBL), polyvalerolactone (PVL), poly(lactic-co-glycolic acid) (PLGA), cellulose acetate propionate (CAP), and combinations thereof.

8. The occlusive device of claim 1, wherein the coiled fiber 102 comprises a diameter from about 0.001 inches to about 0.003 inches.

9. The occlusive device of claim 1, wherein the outer diameter of the coiled configuration of the coiled fiber measures from about 0.008 inches to about 0.018 inches.

10. The occlusive device of claim 1, wherein a majority of the plurality of crossing microfibers each respectively comprises a diameter from about 0.0002 inches to about 0.001 inches.

11. The occlusive device of claim 1, wherein a distal portion of the coiled fiber is configured to form an outer perimeter arrangement within an aneurysm and wherein a proximal portion of the coiled fiber is configured to form an interior array within the outer perimeter arrangement.

12. An occlusive device comprising:
a fiber in a coiled configuration ("coiled fiber");
a stretch-resistant member positioned within a coiled fiber lumen of the coiled fiber; and
a plurality of crossing microfibers securely attached to the stretch-resistant member and extending radially from an outer diameter of the coiled fiber.

13. The occlusive device of claim 12,
wherein the plurality of crossing microfibers extend radially from the coiled fiber across a proximal portion of the coiled fiber,
wherein the plurality of crossing microfibers are absent from a distal portion of the coiled fiber, and
wherein the proximal portion comprises a length measuring from about one half to one third of a total length of the coiled fiber.

14. The occlusive device of claim 13, wherein the distal portion of the coiled fiber is configured to form an outer perimeter arrangement within an aneurysm and wherein the proximal portion of the coiled fiber is configured to form an interior array within the outer perimeter arrangement.

15. The occlusive device of claim 1 or claim 12, wherein materials of the occlusive device consist of bioabsorbable materials.

16. The occlusive device of claim 12,
wherein the coiled fiber comprises a first bioabsorbable material composition comprising a first absorption rate,
wherein the plurality of crossing microfibers comprise a second bioabsorbable material comprising a second absorption rate,
wherein the stretch-resistant member comprises a third bioabsorbable material composition comprising a third absorption rate, and
wherein the second absorption rate is higher than the first absorption rate and the third absorption rate.
